# EUROPEAN PATENT APPLICATION

(11) **EP 4 541 362 A1**
(43) Date of publication of application: **23.04.2025**
(21) Application number: 24203339.7
(22) Date of filing: 27.09.2024
(51) Int. Cl.: A61K 35/747, A61K 35/745, A61P 3/04, A61K 31/00

(54) **PROBIOTIC STRAINS FOR USE IN TREAING OBESITY**

(30) Priority: 17.10.2023 IT 202300021603
(71) Applicant: NOOS S.r.l., 00181 Roma (RM) (IT)
(72) Inventor: MORETTI, Mattia, 00128 Roma (IT); BOCCARUSSO, Marco, 21013 Gallarate VA (IT); UBERTI, Francesca, 28838 Stresa VB (IT)
(74) Representative: Di Giovine, Paolo

(57) **Abstract**

The present invention refers to a composition comprising or consisting of, a mixture of at least one probiotic strain belonging to the *Lacticaseibacillus rhamnosus* species, at least one probiotic strain belonging to the *Bifidobacterium bifidum* species, and at least one probiotic strain belonging to the *Bifidobacterium longum subsp infantis* species and the its use for the prevention or treatment of obesity and metabolic syndrome, as well as to pharmaceutical compositions and kits comprising the composition, to a composition for oral use comprising at least one probiotic strain and policosanols.

## Description

### TECNICHAL FIELD OF THE INVENTION

The present invention refers to a composition comprising or consisting of, a mixture of at least one probiotic strain belonging to the *Lacticaseibacillus rhamnosus* species, at least one probiotic strain belonging to the *Bifidobacterium bifidum* species, and at least one probiotic strain belonging to the *Bifidobacterium longum subsp infantis* species and its use for the prevention or treatment of obesity and metabolic syndrome, as well as to pharmaceutical compositions and kits comprising the composition, to a composition for oral use comprising at least one probiotic strain and policosanols.

### STATE OF THE ART

Obesity is a disease characterized by a pathological accumulation of body fat with consequences also important for the state of health and quality of life.

In recent decades, the incidence of obesity and related diseases has increased throughout the world, affecting more and more children, adolescents and adults.

Childhood obesity has reached epidemic levels not only in developed countries but also in developing ones, with a profound impact on the psychophysical health of children themselves. Several studies have documented that childhood overweight and obesity conditions are associated with long-term adverse outcomes, as factors predictive of an increased mortality rate, mainly due to an increased risk of cardiovascular disease in adulthood; furthermore, obesity in childhood is predictive for the development of type 2 diabetes mellitus.

Obesity is caused, in most cases, by incorrect lifestyle habits characterized by a high-calorie diet and physical inactivity; therefore, from an imbalance between energy intake and consumption. More rarely, however, obesity can be caused by genetic conditions or endocrine diseases, including thyroid dysfunction. Some types of medications can also cause weight gain.

Obesity also represents an important risk factor for hypertension, cardiovascular diseases such as heart attack and stroke, type 2 diabetes mellitus, some forms of tumors, etc.

Obesity on one hand is characterized by an expansion of adipose tissue through a combined increase in the number (hyperplasia) and size (hypertrophy) of adipocyte cells, on the other it is associated with an excessive accumulation of lipids in other tissue cells.

For example, excess lipid deposition in hepatocytes can cause non-alcoholic fatty liver disease (NAFLD), which is the most common chronic liver disease with an incidence paralleling that of obesity.

Metabolic syndrome, also called dysmetabolic syndrome, is not a single and well-defined pathology: it is a pseudo-pathological condition characterized by the concomitance of multiple disorders, such as increased blood sugar, hypercholesterolemia, hypertriglyceridemia and hypertension.

Metabolic syndrome increases the risk of cardiovascular disease, diabetes, stroke and fatty liver disease.

Among the most recurrent etiological elements are: unbalanced diet, insulin resistance (element most involved, responsible for the propensity to diabetes, hypertriglyceridemia, hypertension), obesity, genetic predisposition, sedentary lifestyle.

Metabolic syndrome is a clinical situation in which various interrelated factors contribute to increasing the possibility of developing pathologies affecting the circulatory system and diabetes. For the first time, in 1977, the term metabolic syndrome was used by the german scholar Haller, who associated it with obesity, diabetes mellitus and hepatic steatosis. To date, the exact etiology is not known, but is increasingly considered to be multifactorial in origin. The most frequent associations are represented by overweight and/or obesity, genetic factors, insulin resistance and type 2 diabetes mellitus, sedentary lifestyle and advanced age. A number of markers of systemic inflammation, including C-reactive protein, are often increased, such as fibrinogen, interleukin 6, tumor necrosis factor alpha (TNF-α), and others.

A 2013 clinical study highlighted an increase in the frequency of metabolic syndrome risk factors among subjects with BMI at the upper limit of the normal range.

In the pathogenesis of metabolic syndrome we can identify both exogenous and endogenous factors that interact with each other. Among the exogenous factors, obesity is certainly the main one, even if a sedentary lifestyle and a diet rich in fatty acids and simple sugars contribute to worsening the clinical picture.

Adipose tissue releases non-esterified fatty acids (NEFA, Non-Esterified Fatty Acids), which, if in excess, accumulate in the muscle and liver, favoring insulin resistance and this alteration certainly predisposes to the development of metabolic syndrome. An increase in intracellular fatty acids, or rather in some of their metabolites, contributes to the onset of insulin resistance, hindering the intracellular transmission pathways of the insulin signal. Metabolites accumulate in the form of triglycerides and their increase promotes the production of proinflammatory cytokines.

Furthermore, in subjects with excess weight, adipose tissue is invaded by macrophages; some scholars hypothesize that activated macrophages may increase the production of cytokines, which in turn would be involved in insulin resistance. Visceral adiposity thus becomes the site of a low-intensity chronic inflammatory reaction.

In addition to genetic determinants and unhealthy lifestyle habits, a growing body of scientific evidence from experimental studies and epidemiological evidence indicates that changes in bacterial strains hosted in the human intestine play a key role in obesity.

Indeed, recent studies suggest that the intestinal microbiota is involved in body weight control, energy homeostasis and inflammation, therefore playing a role in the pathophysiology of obesity. The hypothesis that the intestinal microbiota may constitute a relevant environmental factor in the pathogenesis of obesity has led to the study of intestinal microbial communities in overweight subjects, confirming a statistical association between obesity and the peculiar composition of the intestinal microbiota. In fact, the intestinal microbiota plays an important role in the formation and storage of human adipose tissue: intestinal bacteria are able to maintain the energy balance of the human body above all thanks to their ability to share otherwise indigestible components of the mammalian diet. Therefore, an imbalance in this ecosystem can lead to a serious alteration of the intestinal flora, consequently causing weight gain. For example, higher levels of Lactobacillus reuteri and lower levels of Lactobacillus casei/paracasei and Lactobacillus plantarum have been associated with obesity. Numerous works have demonstrated an increase in pro-inflammatory cytokines in such conditions caused by the increased production of endotoxin lipopolysaccharide (LPS), an essential molecule of the cell walls of Gram-negative bacteria that stimulates the deposition of adipose tissue, increasing the degree of inflammation and insulin resistance. However, as emerges from abundant literature, it has been demonstrated that several probiotics, used alone or in symbiotic mixtures, are able to exert their antiobesity effects through species- and strain-specific mechanisms. Therefore, modulation of the intestinal microbiota by probiotics could contribute to reshaping the metabolic profile in the obese.

From the above, it is clear that metabolic syndrome and obesity are closely related.

Therefore, it is essential to contrast lipid dysmetabolism in order to avoid incurring the numerous pathologies resulting from it. The primary objective of metabolic syndrome therapy is the treatment of obesity and different approaches can be followed.

The correction of lifestyles, through the acquisition of correct behaviors both in terms of nutrition and daily physical activity, constitutes the first approach therapy and represents the prevention of secondary diseases. Sometimes, however, especially in patients with various pathologies, it is necessary to carry out pharmacological treatment.

However, the use of pharmacological therapy should not be considered as a substitute for the diet and physical exercise mentioned above; on the contrary, it must be associated with the correction of lifestyle habits.

For the treatment of very severe obesity, bariatric surgery can be used, which has the sole purpose of long-term weight loss with consequent reduced mortality and morbidity. In general, however, malabsorptive interventions are used more frequently rather than solely restrictive ones.

Among the most used drugs to contrast metabolic syndrome we remember those for the treatment of hypertension, to reduce cholesterol, triglycerides and blood sugar.

These drugs also have significant side effects which limit their use to serious situations.

For example, statins can cause, in addition to general side effects (muscle and joint pain, headache, general malaise, gastrointestinal problems and nosebleeds), also more serious problems such as liver damage (with a strong increase in liver enzymes which, if neglected, can cause permanent liver damage), muscle problems (in severe cases, muscle cells can break down and release myoglobin, a protein that can damage the kidneys, into the blood) and diabetes (an increased risk has been found in obese subjects or overweight, already predisposed to the disease).

It is important to point out that there is no drug capable of treating obesity in the absence of adequate correction of eating and lifestyle habits. In fact, the treatment of obesity aims to reduce body weight and the first step to achieve this consists in following an adequate and personalized diet and a regular physical activity program, to be developed on an individual basis.

The main weight loss drugs authorized and on the market are the following:
- Orlistat, a lipase inhibitor drug, indicated as an adjunct to low-calorie diets
- Liraglutide, an active ingredient belonging to the group of GLP-1 receptor analogues. It is used both in the treatment of obesity and overweight and in the treatment of type 2 diabetes mellitus.

The mechanism of action by which liraglutide acts in the treatment of obesity is not yet well defined, but it seems to lead to a reduction in appetite and an increase in sensations of satiety and fullness.

To carry out its action, the active ingredient must be administered parenterally, more precisely, via subcutaneous injection.
- The naltrexone/bupropion combination which appears to act through appetite suppression.

It is important to consider that weight loss drugs or rather drugs for the treatment of obesity/overweight and for weight control should be taken only under medical prescription and under the supervision of the Clinician. It must then be taken into consideration that each drug has specific contraindications and side effects depending on the active ingredient, problems that the doctor will evaluate before prescribing.

The above demonstrates that to contrast these metabolic diseases and syndromes there are products which however have side effects, for example, they cannot be taken long term and must always be prescribed by the doctor who monitors the effect.

Although physical exercise and diet are the first lines of intervention to be recommended, failures or poor results are often recorded. There is currently an increased interest in alternative and effective short-term, non-pharmacological approaches to weight control that involve the use of natural active ingredients.

In recent decades, the prevalence of obesity has reached epidemic proportions, with the number of overweight or obese individuals continuing to increase worldwide. Recent research advances have allowed a better characterization of the etiology of obesity, demonstrating the involvement of the intestinal microbiota.

In fact, if on one hand the signals coming from the brain influence the functionality of the intestine, on the other hand it has been demonstrated that the intestinal microbiota is able to modulate the brain functions involved in the regulation of stress, depression and anxiety, which are closely linked to obesity.

Oral administration of probiotics has been proposed as a valid way to modulate the intestinal ecosystem to promote weight reduction.

Furthermore, there is a felt need for a composition for the treatment of obesity and metabolic syndrome, which has an effect on the parameters of metabolic syndrome that influence obesity, with reduced side effects.

### SUMMARY OF THE INVENTION

The technical problem posed and solved by the present invention is to provide a composition based on different species of probiotics which shows an unexpectedly enhanced effect compared to each species used individually on the parameters of the metabolic syndrome that influence obesity. Furthermore, the composition of the present invention can be taken long-term without side effects.

Furthermore, the composition of the present invention appears to have an improved effect on the parameters of metabolic syndrome that influence obesity when combined with policosanols, showing synergistic activity on the parameters studied.

The data obtained from preliminary tests on single probiotics and their combination were used to evaluate their action on the intestinal barrier. In this context, an in vitro intestinal model was created with CaCo-2 cells which was then stimulated with the substances under investigation. Safety tests and analyzes of barrier integrity were therefore carried out to avoid irritability following the use of probiotics, also evaluating the active role of their metabolites in crossing the intestine.

Considering the potential beneficial effects of probiotics found at the enteric level, the basolateral content metabolised by intestinal cells was then used to disrupt adipogenesis and restore correct lipid metabolism, using a model validated in the literature of adipocyte cells. Specifically, these cells were made to differentiate for 8 days and, during the differentiation process, the cells were stimulated with the metabolites of probiotics produced at the intestinal level, to evaluate the slowing of adipogenesis and the hypolipidemic effect. For this reason, the accumulation of lipids, the measurement of intracellular triglycerides, the intracellular pathways involved in lipid accumulation and lipolysis, the inflammatory picture induced by the accumulation of lipids and the consequent lipid peroxidation were analysed; these parameters represent key conditions to evaluate the role of probiotics, taken orally, in modulating the perpetual increase in body fat and the dysfunction of mature adipocytes.

Finally, the role of the liver following the increase in body fat was also evaluated, since the hepatic accumulation of lipids can promote systemic metabolic dysfunctions; for this reason, HepG2 cells pretreated with oleic acid for 48 hours were used to mimic a condition of metabolic dysfunction in obese subjects. These cells were treated with the metabolites of probiotics produced in the intestine to analyze the reduction of hepatic lipid droplets, the measurement of intracellular triglycerides and the intracellular pathways involved in lipid metabolism.

Specifically, the following surprising and unexpected results were obtained:
- The association of the various selected probiotics is significantly more effective than the same ones used individually on the parameters of the metabolic syndrome that influence obesity;
- The addition of policosanols brought further benefits to these results, especially in the evaluation of adipocyte well-being and in the prevention of oxidative damage;
- All tested substances were able to maintain a correct intestinal function;
- The addition of oil to the formulation with the three probiotics showed an increase in intestinal ionic flow with an increase in the production of second messengers which enter the bloodstream and arrive directly at the target site, showing that the solvent exerts a further improving effect in modulating absorption;
- Following intestinal passage, all the tested substances were able to slow down adipogenesis and modulate the accumulation of triglycerides in adipocytes, suggesting that the use of probiotic-based formulations can produce second messengers capable of modulating the mechanisms inflammatory following lipid accumulation;
- Relevant data were also found at the hepatic level since the conditions of lipid accumulation (mimicked in vitro with oleic acid) were remodulated following the use of the product metabolised at the intestinal level.

The advantages involved are the following:
- possibility of using safe and effective substances, such as probiotics and policosanols, for the treatment of an important problem such as obesity and metabolic syndrome which represent relevant problems for the development of concomitant and related pathologies;
- possibility of using a single product with proven effectiveness:
- possibility of using a synergistic composition that has better activity than its individual components observed in all biological markers to combat obesity and metabolic syndrome.

Therefore, objects of the present invention are:
a composition for oral use comprising, or consisting of, a mixture of at least one probiotic strain belonging to the *Lacticaseibacillus rhamnosus* species, at least one probiotic strain belonging to the *Bifidobacterium bifidum* species, and at least one probiotic strain belonging to the *Bifidobacterium longum subsp infantis* species;
a pharmaceutical composition, dietary supplement, medical device, food for special medical purposes, or functional food comprising or consisting of the composition according to any of the embodiments described herein;
a composition for oral use comprising at least one probiotic strain and policosanols, for use in the prevention or treatment of obesity and/or metabolic syndrome, preferably said probiotic strain belonging to the species *Lacticaseibacillus rhamnosus, Bifidobacterium bifidum or Bifidobacterium longum subsp infantis;*
a kit of parts comprising a mixture of at least one probiotic strain belonging to the species Lacticaseibacillus rhamnosus, at least one probiotic strain belonging to the species Bifidobacterium bifidum, and at least one probiotic strain belonging to the species Bifidobacterium longum subsp infantis as defined in any of the described embodiments below, and policosanols.

Further advantages and/or embodiments of the present invention will be evident from the following detailed description.

### DETAILED DESCRIPTION OF THE FIGURES

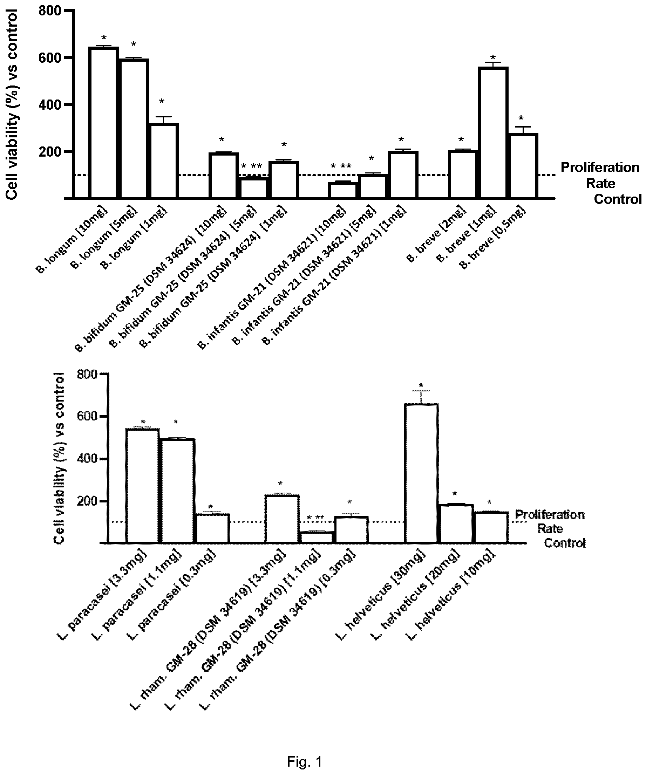

**Fig. 1** **Screening of probiotic strains.** Dose-response study on 3T3-L1 cells. Results are expressed as mean ± SD (%) of 5 biological replicates normalized to the control. * p<0.05 vs control; **p<0.05 vs other concentrations. A cell viability analysis showing data above 100% presupposes an increase in cell proliferation. Some of the tested strains significantly exceed the selected cut-off (>100%) (p<0.05). Only B. bifidum GM-25 DSM 34624 at 5mg, B. infantis GM-21 DSM 34621 at 10mg and L. rhamnosus GM-28 DSM 34619 at 1.1mg are able to maintain cell viability above control (untreated cells) but below the proliferation cut-off, suggesting that these substances do not stimulate adipocyte production.

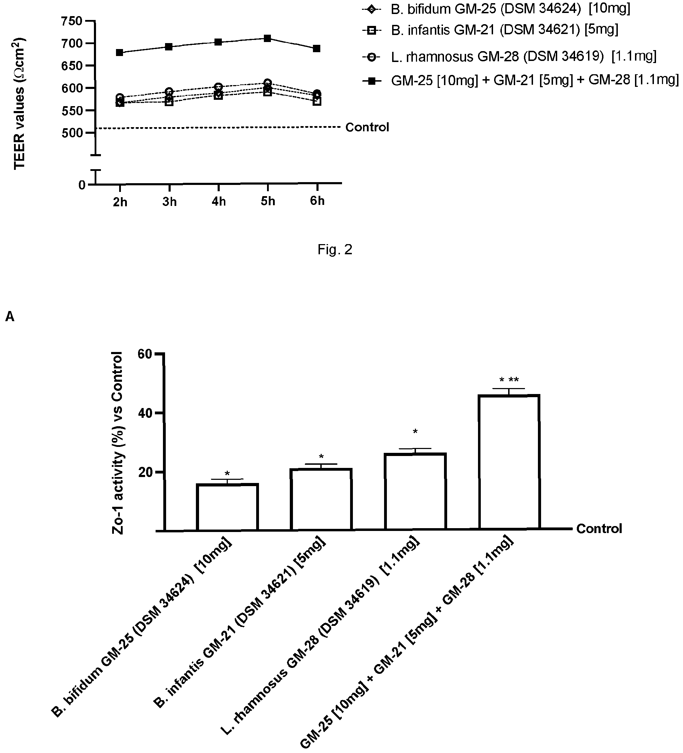

**Fig. 2** **Evaluation of intestinal barrier permeability. Trans Epithelial Resistance (TEER).** Test results are expressed as mean ± SD (%) of 5 biological replicates normalized to the control. p<0.05 vs control. All tested samples were able to maintain epithelial integrity by increasing the ion flux of paracellular exchanges across the intestinal epithelium compared to the control (p<0.05). The association of the three strains showed better activity.

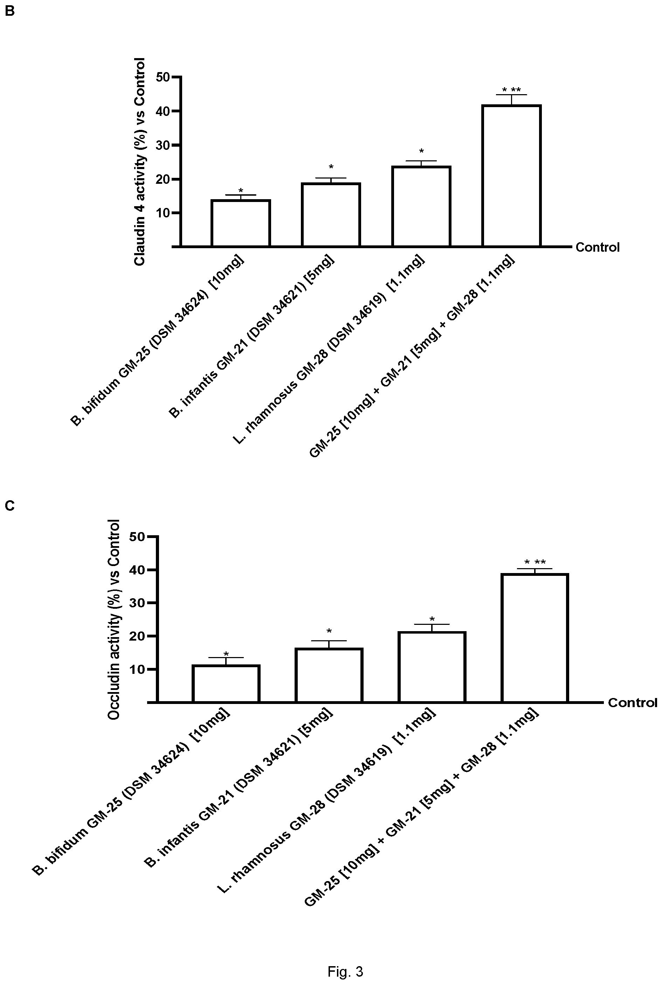

**Fig. 3** **Analysis of TJ activity using ELISA Kit (Zonulin, claudin and occludin).** In (A) analysis of ZO-1; in (B) analysis of Claudina 4; in (C) Occludin analysis. Test results are expressed as mean ± SD (%) of 5 biological replicates normalized to the control. * p < 0.05 vs control, ** p < 0.05 vs single agents. The analyzes obtained support the hypothesis of the importance of the formulation made up of the three probiotic strains to induce greater absorption associated with increases in intestinal barrier functions mediated by TJ.

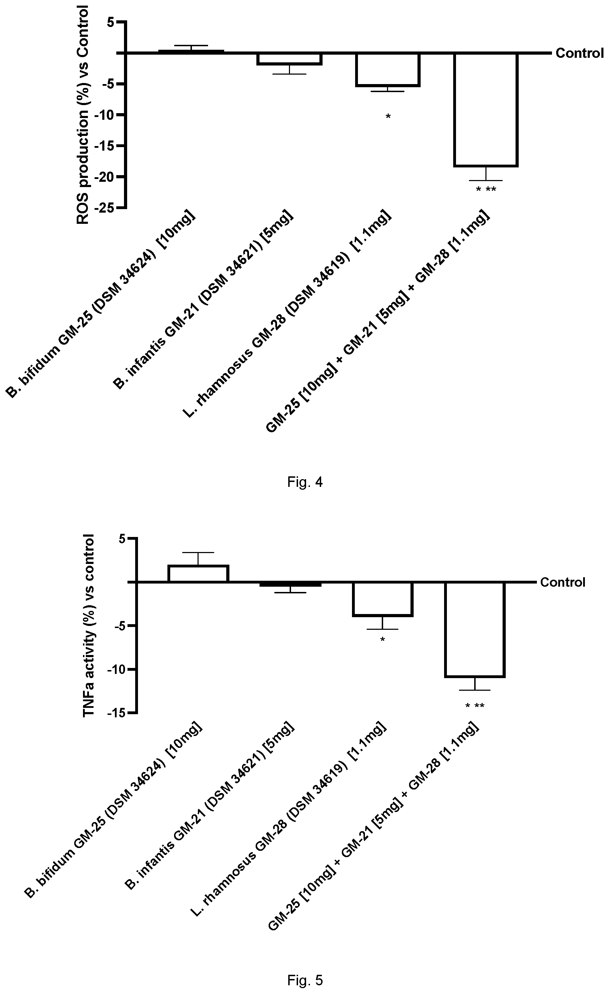

**Fig. 4** **Oxidative stress. Analysis of ROS production.** Test results are expressed as mean ± SD (%) of 5 biological replicates normalized to the control. * p < 0.05 vs control, ** p < 0.05 vs single agents. The analyzes conducted demonstrate that the formulations reduce the production of ROS compared to the control (p<0.05) and compared to the individual substances, suggesting the absence of oxidative stress at the level of the intestinal barrier.
**Fig. 5** **Analysis of TNFα activity.** Test results are expressed as mean ± SD (%) of 5 biological replicates normalized to the control. * p < 0.05 vs control, ** p < 0.05 vs single agents. The data on TNFα support the hypothesis of lack of intestinal inflammation, already indicated by the reduction in ROS production.

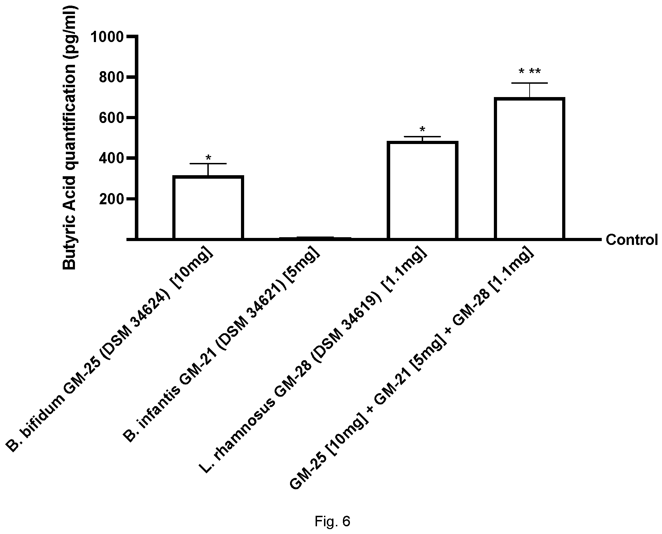

**Fig. 6** **Secondary metabolites (butyric acid).** Test results are expressed as mean ± SD (%) of 5 biological replicates normalized to the control. * p<0.05 vs control, ** p<0.05 vs individual strains. The analysis of butyric acid shows that the combination of probiotics is able to stimulate the production of second messengers in the bloodstream in greater quantities than the individual strains.

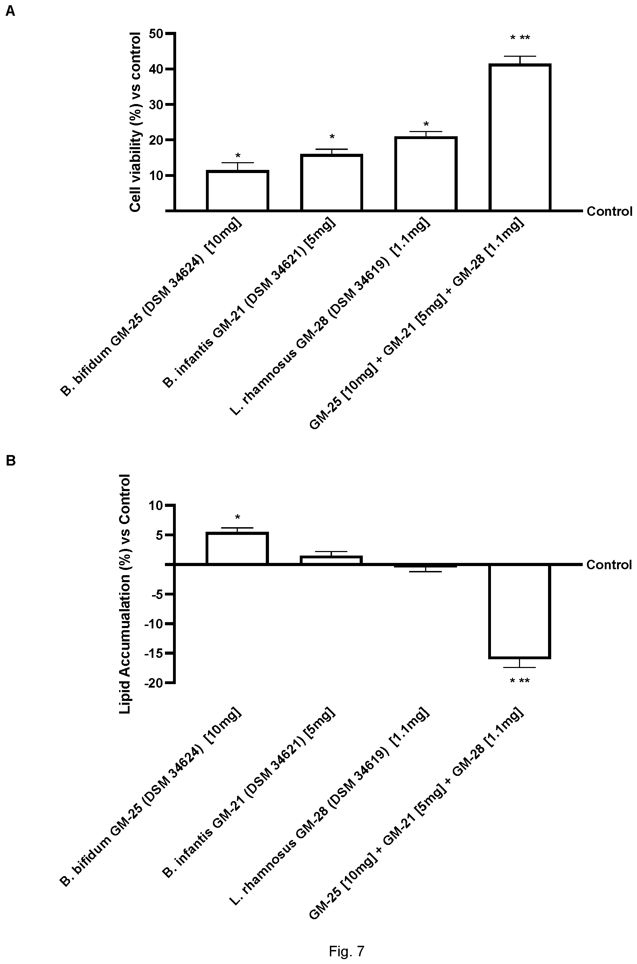

**Fig. 7** **Safety and efficacy on adipocytes.** In (A) determination of cell viability; in (B) analysis of lipid accumulation. Test results are expressed as mean ± SD (%) of 5 biological replicates normalized to the control. * p < 0.05 vs control, ** p < 0.05 vs individual strains. The tested strains induce an increase in cell viability compared to the control (p<0.05). These data confirm that the use of the probiotic combination is able to reduce lipid accumulation at the adipocyte level.

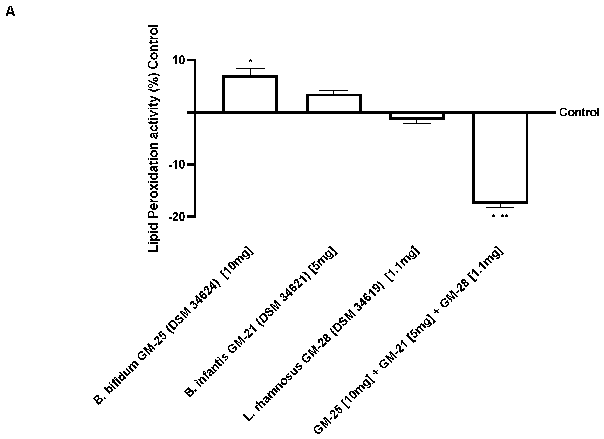

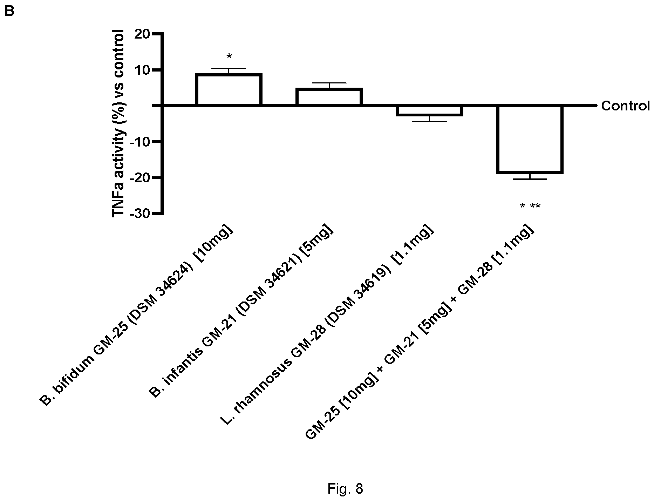

**Fig. 8** **Lipid peroxidation of adipocytes.** In (A) lipid peroxidation analysis; in (B) analysis of TNFα activity. Test results are expressed as mean ± SD (%) of 5 biological replicates normalized to the control. * p < 0.05 vs control, ** p < 0.05 vs individual strains. The analysis relating to oxidative stress (lipid peroxidation) suggests the absence of antioxidant mechanisms following stimulation with the association of the tested strains, confirming the positive role of probiotics in modulating adipogenesis. The analysis on TNFa also confirms the absence of inflammatory processes following treatment with the new formulations.

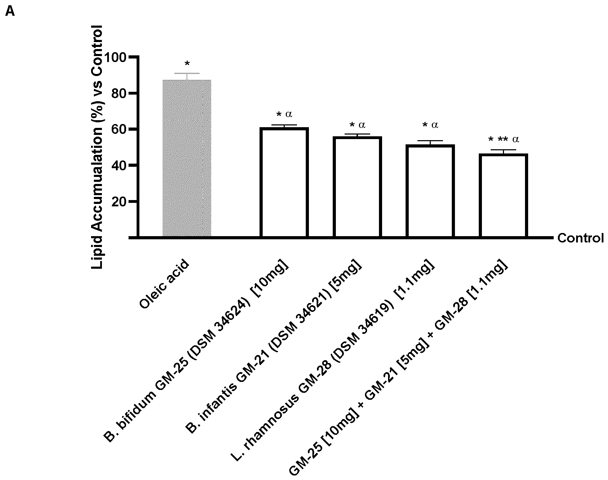

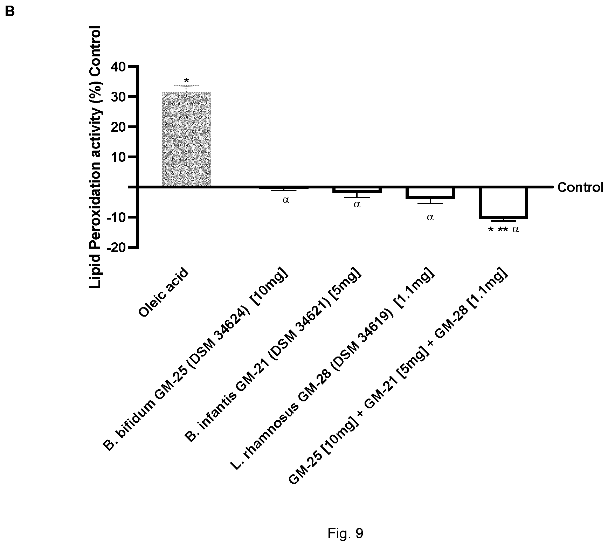

**Fig. 9** **Liver tissue. Lipid accumulation.** In (A) analysis of lipid accumulation; in (B) lipid peroxidation analysis. Test results are expressed as mean ± SD (%) of 5 biological replicates normalized to the control. * p < 0.05 vs control, ** p < 0.05 vs individual strains, α p < 0.05 vs oleic acid. The strains reduce lipid accumulation without inducing oxidative stress in the liver. Specifically, the combination of probiotics further reduces this effect (p<0.05).

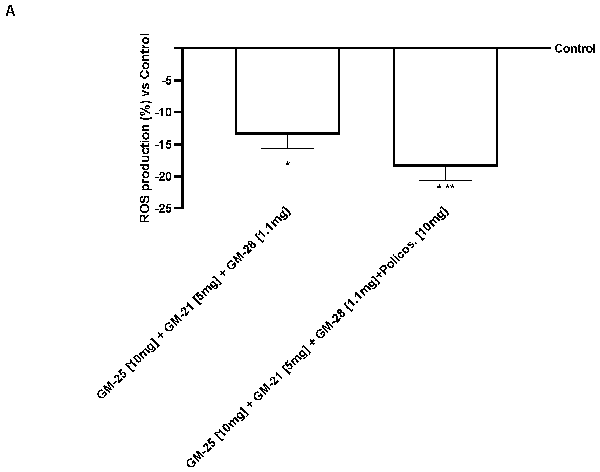

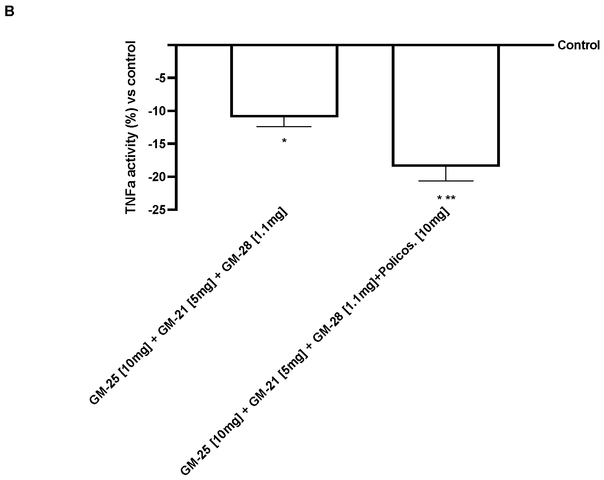

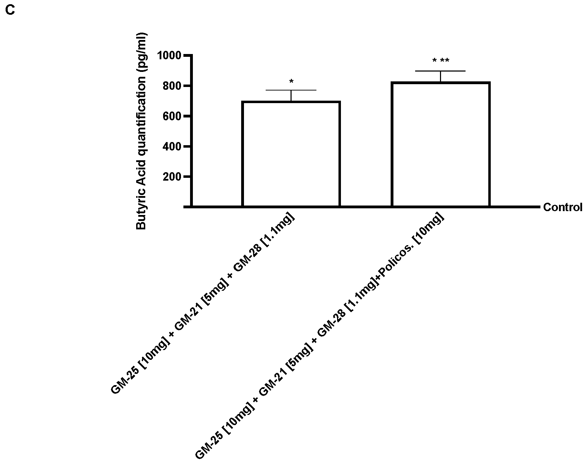

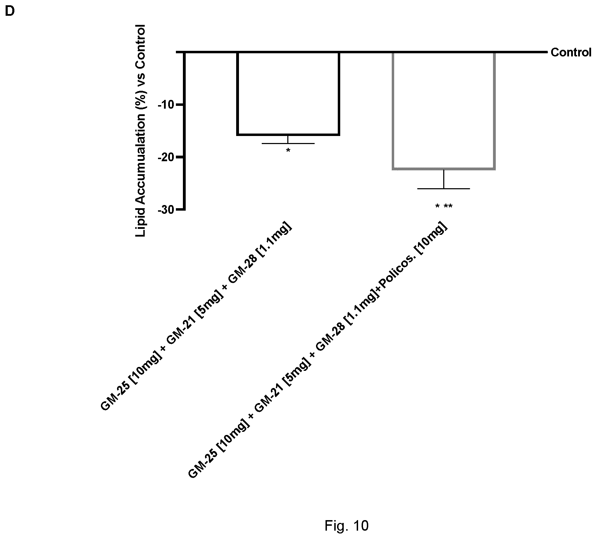

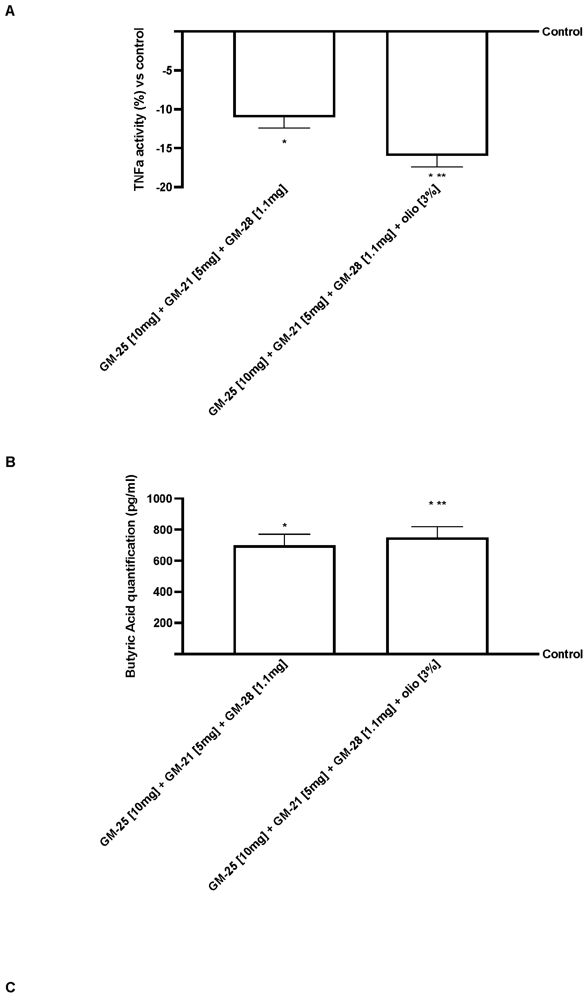

**Fig. 10** **Effect of policosanols.** Analysis of the activity of policosanols on A) ROS; B) TNF alpha; C) Secondary metabolites (Butyric acid); D) Lipid accumulation. The tests were conducted with the same methods used in previous studies. It is noted that the addition of policosanols to the mixture of probiotic strains brings an improvement in the response to all the parameters examined. Test results are expressed as mean ± SD (%) of 5 biological replicates normalized to the control. * p < 0.05 vs control, ** p < 0.05 association containing policosanols compared to probiotic combination alone.

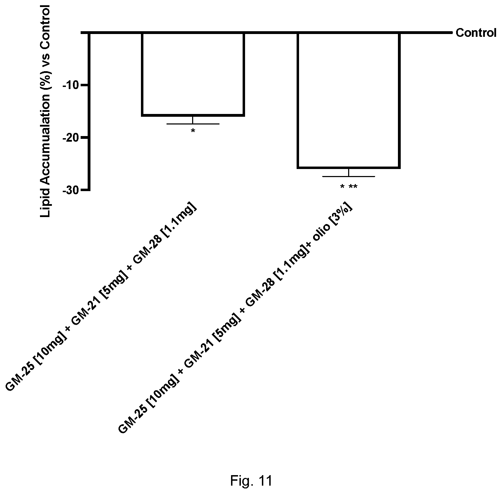

**Fig. 11** **Effect of oil.** Analysis of the activity of vegetable oil on A) TNF alpha; B) Secondary metabolites (Butyric acid); C) Lipid accumulation. The tests were conducted with the same methods used in previous studies. It is noted that the addition of the oil to the mixture of probiotic strains brings an improvement in the response to all the parameters examined. Test results are expressed as mean ± SD (%) of 5 biological replicates normalized to the control. * p < 0.05 vs control, ** p < 0.05 oil-containing combination compared to probiotic combination alone.

### GLOSSARY

### Probiotics

According to the official definition of FAO and WHO, probiotics are "live micro-organisms which, administered in adequate quantities, bring a benefit to the health of the host".

It should be underlined that not all probiotics are effective in combating obesity and metabolic disorders. You should look for specific strains with these characteristics.

### Lactobacilli

Lactobacilli are a genus of bacteria with a rod-like structure, Gram-positive, facultative anaerobes or microaerophiles. They constitute the majority of the group of lactic acid bacteria, so called because almost all of their members convert lactose and other sugars into lactic acid through lactic fermentation. They are very common and usually non-pathogenic. In the human organism they are present in the vagina and gastrointestinal tract, where they are symbiotic and constitute a small part of the human microbiota.

They perform various functions useful for human health. For example, they can contribute to the digestion of food, the absorption of nutrients and hold off pathogenic microorganisms that can trigger problems such as diarrhea.

They can be taken in the form of foods (especially yogurt and other fermented milks) or food supplements.

### Bifidobacteria

Bifidobacteria are among the dominant bacterial populations in the human gastrointestinal tract. They are pleomorphic, non-spore-forming, Gram-positive bacilli, obligate anaerobes but sometimes aerotolerant, chemorganotrophic, immobile. Bifidobacteria are saccharolytic organisms and have the ability to ferment glucose, galactose and fructose. Glucose is fermented via the fructose-6-phosphate shunt into acetic and lactic acid.

### Policosanols

Policosanols are natural substances that are extracted from sugar cane and some by-products of cereal processing (wheat germs, rice wax). From a chemical point of view, it is a mixture of long-chain linear aliphatic alcohols (octacosanol, tetracosanol, hexacosanol and others).

In the present description, the unit of measurement "CFU/g" refers to "Colony Forming Units per gram", which in Italian means "Colony Forming Units per gram". This is a unit of measurement commonly used in microbiology to quantify the number of colony-forming units (colons) of microorganisms present in a sample of substance (usually in a biological sample or in an environmental sample) per unit of weight, which is in this case the gram.

### DETAILED DESCRIPTION

The present invention refers to a composition for oral use comprising, or consisting of, a mixture of at least one probiotic strain belonging to the *Lacticaseibacillus rhamnosus* species, at least one probiotic strain belonging to the *Bifidobacterium bifidum* species, and at least one probiotic strain belonging to the *Bifidobacterium longum subsp infantis* species.

In one embodiment, said probiotic strain belonging to the *Lacticaseibacillus rhamnosus* species is the *Lacticaseibacillus rhamnosus* strain GM-28 DSM 34619, filed at the German Collection of Microorganisms and Cell Cultures GmbH; and/or
said probiotic strain belonging to the *Bifidobacterium bifidum* species is the *Bifidobacterium bifidum* strain GM-25 DSM 34624, filed at the German Collection of Microorganisms and Cell Cultures GmbH; and/or
said probiotic strain belonging to the species *Bifidobacterium longum subsp infantis* is the strain *Bifidobacterium longum subsp infantis* GM-21 DSM 34621 filed at the German Collection of Microorganisms and Cell Cultures GmbH.

### Lacticaseibacillus rhamnosus GM-28 DSM 34619

The morphology of the cells of the L. rhamnosus GM-28 strain is rod-shaped, they can be found in single conformations or in chains of different units.

Strain GM-28 has been genetically identified as *Lacticaseibacillus rhamnosus* and the species *Lacticaseibacillus rhamnosus* is recognized with QPS qualification according to EFSA.

In 2020, the Lactobacilli genus underwent a taxonomic reclassification, whereby the many species that belonged to it were relocated to new genera.

Lacticaseibacillus is one of these new genera.

*Lacticaseibacillus rhamnosus* is the correct name used since 2020 to refer to the species previously known as Lactobacillus rhamnosus.

*Lacticaseibacillus rhamnosus* GM-28 was filed at the German Collection of Microorganisms (DSMZ) on 05/16/2023 by the depositor Nóos Srl, with accession number DSM 34619.

Genetic identification at the species and strain level for *Lacticaseibacillus rhamnosus* GM-28 was carried out by gene amplification with the polymerase chain reaction (PCR) technique, sequencing and investigation of the NCBI GenBank database.

### Bifidobacterium bifidum GM-25 DSM 34624

The morphology of the cells of the *B. bifidum* GM-25 strain is curved and irregular in shape, often in a bifid Y or V conformation.

Strain GM-25 has been genetically identified as *Bifidobacterium bifidum* and the *Bifidobacterium bifidum* species is recognized with QPS qualification according to EFSA.

*Bifidobacterium bifidum* GM-25 was filed at the German Collection of Microorganisms (DSMZ) on 05/16/2023 by the depositor Nóos Srl, with accession number DSM 34624.

Genetic identification at the species and strain level for *Bifidobacterium bifidum* GM-25 was carried out by gene amplification with the polymerase chain reaction (PCR) technique, sequencing and investigation of the NCBI GenBank database.

### Bifidobacterium longum subsp infantis GM-21 DSM 34621

The cell morphology of B. *infantis* strain GM-21 is irregular and bifid. The B. *infantis* GM-21 strain is obligately anaerobic.

Strain GM-21 was genetically identified as Bifidobacterium longum subsp. infantis and the Bifidobacterium longum species is recognized with the QPS qualification according to the EFSA.

*Bifidobacterium longum subsp. infantis* GM-21 was filed at the German Collection of Microorganisms (DSMZ) on 05/16/2023 by the depositor Nóos Srl, with accession number DSM 34621.

Genetic identification at the species and strain level for *Bifidobacterium longum subsp. infantis* GM-21 was performed by gene amplification with the polymerase chain reaction (PCR) technique, sequencing and investigation of the NCBI GenBank database.

The bioinformatic analysis of the strains confirmed that they are original strains, which have no traces of genetic modifications. It is important to underline that they were found to be safe as they do not show virulence factors or genetic resistance elements to antibiotics.

Evaluation of all strains for transferable antibiotic resistance was carried out by determining the minimum inhibitory concentration (MIC) using a panel of antibiotics based on EFSA guidance. All MIC values determined for all strains analyzed are lower than the values defined by EFSA for the *Lactobacillus acidophilus* and *Bifidobacterium* groups. Phenotypic characterization therefore confirmed all strains are susceptible to antibiotics relevant for their use in humans and animals.

In conclusion, no dangers and therefore risks for consumers and for the environment have been identified arising from the consumption or use of *Lacticaseibacillus rhamnosus* GM-28, *Bifidobacterium longum subsp. infantis* GM-21 and *Bifidobacterium bifidum* GM-25.

Given this, these strains can be considered safe for human consumption and comply with the requirements established by the Italian Ministry of Health for probiotics to be used in foods and food supplements.

In one embodiment, said probiotic strain belonging to the species *Lacticaseibacillus rhamnosus, Bifidobacterium bifidum* and/or *Bifidobacterium longum subsp infantis* is present in a concentration between 1×10⁷ and 5×10¹¹ CFU/g, preferably between 1×10⁸ and 1×10¹⁰ CFU/g.

With CFU/g in the present invention, reference is made to the number of colony forming units (CFU), relative to one gram of composition.

In one preferred embodiment, said probiotic strains belonging to the species *Lacticaseibacillus rhamnosus, Bifidobacterium bifidum* and *Bifidobacterium longum subsp infantis* are present in a concentration between 1×10⁷ and 5×10¹¹ CFU/g, preferably between 1×10⁸ and 1×10¹⁰ CFU/g. In one preferred embodiment, said probiotic strain belonging to the *Lacticaseibacillus rhamnosus* species is the *Lacticaseibacillus rhamnosus* strain GM-28 DSM 34619,
said probiotic strain belonging to the *Bifidobacterium bifidum* species is the *Bifidobacterium bifidum* strain GM-25 DSM 34624, and
said probiotic strain belonging to the species Bifidobacterium longum subsp infantis is the strain *Bifidobacterium longum subsp infantis* GM-21 DSM 34621,
and are present in a concentration between 1×10⁷ and 5×10¹¹ CFU/g, preferably between 1×10⁸ and 1×10¹⁰ CFU/g.

In one embodiment, the total probiotic concentration is between 5×10⁷ and 5×10¹² CFU/g, preferably 5×10⁸ and 5×10¹⁰ CFU/g. By total concentration of probiotics it is meant the sum of the concentrations of the probiotic strain belonging to the *Lacticaseibacillus rhamnosus* species, of the probiotic strain belonging to the *Bifidobacterium bifidum* species, and of the probiotic strain belonging to the *Bifidobacterium longum subsp infantis* species.

From the results obtained in the experimental section reported in the following paragraphs, relating to tests on probiotics taken individually and their combination, it appears that the composition of the present invention shows an enhanced effect compared to each strain species used individually, on the parameters of the metabolic syndrome that influence obesity in a 3D model by seeding CaCo-2 intestinal cells into the apical part of a Transwell^{®} system, which was then stimulated with the tested substances.

The parameters of the metabolic syndrome that influence obesity that have been considered are the accumulation of lipids, the measurement of intracellular triglycerides, the intracellular pathways involved in lipid accumulation and lipolysis, the inflammatory framework induced by the accumulation of lipids and by the resulting lipid peroxidation, as well as safety testing and barrier integrity analysis.

### Mechanism of action of probiotics for weight reduction

It is known that changes in the structure of the microbiota caused by external agents can cause the development of metabolic diseases. Even without linking the present invention to any specific mechanism of action, the following considerations are reported.

A non-limiting example of the mechanism of action for probiotic-mediated weight reduction includes:
1. Modulation of the intestinal microbiota and production of short-chain fatty acids (SCFA), which regulate fat metabolism. This process increases the "fat burning" effect and reduces the accumulation of fat.
2. An altered microbiota can facilitate the extraction of energy from the diet, generating an excess of calories with consequent accumulation.
3. Regulation of energy homeostasis and/or satiety.
4. Antagonistic effect against the growth of pathogenic microorganisms and competitive adherence to the intestinal mucosa (antibacterial activity).
5. Increased production of the intestinal mucus layer and reduction of intestinal permeability, thus performing a barrier function for the intestine.
6. Modulation of the gastrointestinal immune system (immunological activity).
7. The intestinal microbiota is able to influence brain functions involved in the regulation of stress, depression and anxiety, all closely linked to obesity. Probiotics play an important role in restoring body weight balance and supporting weight control efforts during dieting, thanks to their action on the gut-brain axis.
8. Reduction of intestinal inflammation associated with obesity.

These mechanisms reflect the important role of probiotics in promoting weight loss and body weight maintenance through a variety of interconnected processes.

Surprisingly, the Authors of the present invention also discovered that the composition of the present invention appears to have an improved effect on metabolic syndrome parameters that influence obesity when combined with policosanols, showing further synergy on the studied parameters.

Therefore, in one embodiment, the composition according to any of the embodiments described herein further comprises policosanols.

Policosanols can be obtained from a hydroalcoholic extract of cereals, rice or sugar cane. In a preferred embodiment said policosanols are in the form of an extract obtained from rice. Preferably, said extract is in a concentration of policosanols at 98% by weight with respect to the total weight of the extract.

In a preferred embodiment, said policosanols have a concentration in octacosanols of at least 50%, preferably at least 60% by weight with respect to the total weight of policosanols.

An example of a commercial product used that contains policosanols is POLICOSANOL 98% (60% OCTACOSANOL) by Vivatis Pharma.

In one preferred embodiment, said policosanols are present in a concentration of from 0.1% to 80% by weight with respect to the total weight of the composition, preferably from 0.5% to 25% by weight with respect to the total weight of the composition.

In one preferred embodiment, the dose of policosanols ranges from 1 to 100 mg per daily dose, preferably 10 mg/day.

The authors of the present invention have also surprisingly discovered that by using a vegetable oil as a solvent in the composition object of the present invention, the resulting composition shows an increase in intestinal ionic flow with an increase in the production of second messengers which enter the bloodstream and arrive directly to the target site, thus leading to a further improving effect in modulating intestinal absorption.

Therefore, in one embodiment, the composition further comprises a vegetable or synthetic oil, preferably selected from olive oil, sunflower oil, corn oil, linseed oil, hemp oil or mixtures thereof.

In a preferred embodiment, preferably a mixture of hemp oil and linseed oil in a weight ratio of 80:20.

In one embodiment, said vegetable oil is present in a concentration of 1% to 20%, preferably 1 to 10%, even more preferably 3% by weight with respect to the total weight of the composition.

In light of the surprising improved effects of the probiotic composition of the present invention compared to the effect of individual probiotic strains on metabolic syndrome parameters that influence obesity such as lipid accumulation, measurement of intracellular triglycerides, the intracellular pathways involved in lipid accumulation and lipolysis, the inflammatory picture induced by lipid accumulation and the consequent lipid peroxidation, as well as the safety and integrity of the barrier, it is advantageous to use the composition of the present invention in a pharmaceutical composition, food supplement, device medical, food for special medical purposes or functional food.

Therefore, the object of the present invention is also a pharmaceutical composition, food supplement, medical device, food for special medical purposes or functional food comprising or consisting of the composition according to any of the embodiments described here.

Depending on the type of formulation, the composition can further comprise at least one pharmaceutically and/or nutraceutically acceptable excipient and/or carrier.

In a preferred embodiment, the pharmaceutical composition of the invention is in the form of a powder in sachets, sticks, tablets, capsules or drops.

Pharmaceutically acceptable excipients and carriers suitable for the compositions of the present invention are known to those skilled in the art.

Food supplements are food products intended to supplement the common diet and which constitute a concentrated source of nutrients, such as vitamins and minerals, or other substances having a nutritional or physiological effect, or probiotics, both single-compound and multi-compound, in pre-dosed forms.

Medical device, in the present invention, corresponds to a medical device according to any of the classes described in the EU Regulation 2017/745 on medical devices (which also includes substances and not just "devices" in the mechanical sense of the term).

By functional food, in the present invention, it is meant a food which, beyond its basic nutritional properties, has a demonstrated ability to positively influence one or more physiological functions. A fundamental prerogative is also to contribute to preserving or improving the state of health and/or reducing the risk of the onset of diseases related to the diet.

In one embodiment, the composition, pharmaceutical composition, dietary supplement, medical device, food for special medical purposes or functional food of the present invention is for use in the prevention or treatment of obesity and/or metabolic syndrome.

The composition, pharmaceutical composition, food supplement, medical device, food for special medical purposes or functional food, according to any of the embodiments described herein, may be administered with a dosage in which the probiotic strain belonging to the species Lacticaseibacillus rhamnosus, *Bifidobacterium bifidum* and/or Bifidobacterium longum subsp infantis is between 1×10⁷ and 1×10¹¹ CFU/daily dose, preferably from 1×10⁸ to 1×10¹⁰ CFU/daily dose.

In a preferred embodiment, the total dosage of probiotics is between 5×10⁷ and 5×10¹¹ CFU/daily dose, preferably from 5×10⁸ to 5×10¹⁰ CFU/daily dose.

A further object of the present invention is a composition for oral use comprising at least one probiotic strain and policosanols, for use in the prevention or treatment of obesity and/or metabolic syndrome.

In one preferred embodiment, said probiotic strain belongs to the species Lacticaseibacillus rhamnosus, *Bifidobacterium bifidum* and/or Bifidobacterium longum subsp infantis.

In one even more preferred embodiment, said probiotic strain belonging to the *Lacticaseibacillus rhamnosus* species is the *Lacticaseibacillus rhamnosus* strain GM-28 DSM 34619, filed at the German Collection of Microorganisms and Cell Cultures GmbH; and/or
said probiotic strain belonging to the *Bifidobacterium bifidum* species is the *Bifidobacterium bifidum* strain GM-25 DSM 34624, filed at the German Collection of Microorganisms and Cell Cultures GmbH; and/or
said probiotic strain belonging to the species Bifidobacterium longum subsp infantis is the strain Bifidobacterium longum subsp infantis GM-21 DSM 34621 filed at the German Collection of Microorganisms and Cell Cultures GmbH.

In one preferred embodiment, the composition also includes vegetable or synthetic oil preferably selected from olive oil, corn oil, sunflower oil, hemp oil, linseed oil or mixtures thereof.

In a one preferred embodiment, preferably a mixture of hemp oil and linseed oil in a weight ratio of 80:20.

In one embodiment, said vegetable oil is present in a concentration from 1% to 20%, preferably from 1 to 10%, even more preferably 3% by weight with respect to the total weight of the composition.

A further object of the present invention is a kit of parts comprising a mixture of at least one probiotic strain belonging to the *Lacticaseibacillus rhamnosus* species, at least one probiotic strain belonging to the *Bifidobacterium bifidum* species and at least one probiotic strain belonging to the *Bifidobacterium longum subsp infantis* species as defined in any of the embodiments described herein, and policosanols.

In one embodiment, the kit of the present invention can be for the simultaneous or sequential administration of said strains and said policosanols.

In any part of this description and the claims, the term comprising may be replaced by the term "consisting of".

Below are some examples which have the purpose of better illustrating the methodologies disclosed in this description, these examples are in no way to be considered a limitation of the previous description and the subsequent claims.

### EXPERIMENTATION AND EXAMPLES

### Example1

### Selection of probiotic strains and dose-response evaluation of individual strains

A preliminary screening was carried out following stimulation with some probiotics at different concentrations of 3T3-L1 adipose cells for 24h, the time necessary to verify the absence of any toxic effect.

The strains tested were:
*Bifidobacterium longum* BLG01
*Lactobacillus paracasei* TJB8
*Bifidobacterium brevis* BR500
*Lactobacillus helveticus* TJA4
*Lacticaseibacillus rhamnosus* GM-28 DSM 34619,
*Bifidobacterium bifidum* GM-25 DSM 34624
*Bifidobacterium longum subsp infantis* GM-21 DSM 34621

As highlighted in *Figure 1*, most probiotics, tested at different concentrations, exceed the threshold value of 100% viability, therefore indicating an increase in cell proliferation. In particular, all tested concentrations of B. longum, L. paracasei, B. Breve and L. helveticus induce an increase in cell proliferation (p<0.05), leading to an increase in the number of adipocytes; therefore these strains were not used for subsequent analyses.

As regards the other probiotics tested, only B. bifidum GM-25 DSM 34624 5mg (corresponding to 0.5 × 109 CFU), B. infantis GM-21 DSM 3462110mg (corresponding to 1 × 109 CFU) and L. rhamnosus GM -28 DSM 346191.1mg (corresponding to 0.33 × 109 CFU) are able to maintain cell viability above the control level but below the proliferation cut-off value (p<0.05), therefore suggesting that these probiotics do not stimulate the production of adipocytes.

On the basis of these results, the three indicated probiotics *Lacticaseibacillus rhamnosus* GM-28 DSM 34619, *Bifidobacterium bifidum* GM-25 DSM 34624 and *Bifidobacterium longum subsp infantis* GM-21 DSM 34621 were chosen for the composition of the final product to be used in the concentrations and ratios indicated above

### Example 2

### Evaluation of the Safety (a) and Effectiveness (b - c) at the intestinal level of probiotics, taken individually or in combination with each other

### Safety

### (a) Evaluation of permeability and integrity on an in vitro intestinal barrier model (TEER and TJ activity)

To evaluate and analyze the correct functioning of the intestinal barrier, tests were conducted on a 3D in vitro model to mimic the complexity of the human intestinal barrier. In this context, probiotics alone and in combination were tested over a 2 to 6 hour interval to measure TEER values and TJ activity.

### In vitro intestinal barrier model (TEER) Figure 2

An intestinal barrier model was created, using CaCo-2 cells, to analyze the passage of the test substances through the intestinal barrier. For this reason, TEER values were determined with EVOM3, coupled with STX2 rod electrodes (World Precision Instruments, Sarasota, FL, USA); this assay was performed every 2 days for 21 days until reaching a TEER value ≥ 400 Ωcm2 before stimulation, the time necessary for the formation of the cell monolayer, for cell differentiation and for the exposure of the intestinal villi. On day 21, the culture medium at the apical and basolateral level was changed to create different pH conditions: pH around 6.5 at the apical level (acidic pH mimicking the lumen of the small intestine) and pH around 7.4 at the basolateral level (neutral pH that imitates human blood). The cells were maintained for 15 minutes at 37°C and 5% CO2, after which the TEER values were measured again before the start of the experiment to verify the stabilization of the values. At the end of the stimulation interval, medium from the basolateral environment was collected to be used to subsequently stimulate 3T3-L1 cells and HepG2 cells.

### Tight Junction Activity (Zonulin, Claudin, Occludin) Figure 3 (A, B, C)

Membrane proteins that perform the following functions have been determined:
**ZO1:** maintains the TJ structure and modulates the integrity of the barrier
**Claudine-4:** the main barrier-forming protein
**Occludin:** Contributes to TJ stabilization and optimal barrier function
• *Analysis of ZO-1 activity*

The Human Tight Junction Protein 1 ELISA kit (MyBiosource, San Diego, CA, USA) was measured in CaCo-2, following the manufacturer's instructions. Briefly, cells were washed with cold 1× PBS (Merck Life Science, Rome, Italy) and processed with two freeze/thaw cycles; then, cell lysates were centrifuged for 5 min at 5000 × g at 4°C. After that, 100 µL of each sample was collected and incubated on the ELISA plate at 37 °C for 90 minutes; after washing, 100 µL of detection solution A was added to each well and incubated for 45 minutes at 37 °C. The wells were washed and 100 µL of Detection Solution B was added to the samples. After a 45 minute incubation, the wells were washed again and 90 µL of substrate solution was added to each well, then the samples were incubated for 20 minutes at 37°C in the dark. Finally, 50 µL of stop solution was added and the plates were read on the spectrophotometer (Infinite 200 Pro MPlex, Tecan, Männedorf, Switzerland) at a wavelength of 450 nm. The concentration was expressed as pg/mL, comparing the data with the standard curve (range 0 to 1000 pg/mL), and the results were expressed as percentage (%) compared to the control (line 0). *Figure 3**°*

### • Analysis of Claudine 4 activity

Human Claudine 4 was measured in CaCo-2 lysates by ELISA kit (Cusabio Technology LLC, Huston, TX, USA), following the manufacturer's instructions [18]. Briefly, cells were lysed with cold 1× PBS (Merck Life Science, Rome, Italy) and centrifuged at 1500× g for 10 min at 4°C. Then, 100 µL of each sample was added to the ELISA plate and incubated at 37 °C for 2 h; after which, the plate was washed and 100 µL of biotin antibody was added to the wells and incubated for 1 hour at 37 °C. After this time, the wells were washed and 100 µL of HRP-avidin was added to each well; the samples were incubated for 1h at 37°C. Subsequently, 90 µL of TM B substrate was also added to the samples and the plate was incubated for 20 minutes at 37 °C protected from light. Upon completion, 50 µL of stop solution was used to stop the reaction and the plate was analyzed with a spectrophotometer (Infinite 200 Pro MPlex, Tecan, Männedorf, Switzerland) at 450 nm. The concentration was expressed as pg/mL, comparing the data with the standard curve (range 0 to 1000 pg/mL), and the results were expressed as percentage (%) compared to the control (line 0). *Figure 3B*

### • Occludin activity analysis

The human occludin ELISA kit (OCLN kit, MyBiosource, San Diego, CA, USA) analyzed the presence of occludin in CaCo-2 cell lysates, according to the manufacturer's instructions. Briefly, CaCo-2 cells were lysed with Phosphate-Buffered Saline (PBS, Merck Life Science, Rome, Italy) 1×, centrifuged at 1500× g for 10 min at 4 °C, and 100 µL of each sample was transferred to the strip well prior to incubation at 37°C for 90 minutes. The supernatants were removed and the strips were incubated with 100 µL of detection solution A for 45 minutes at 37 °C; then, the strips were washed with the washing solution and incubated with 100 µL of detection solution B for another 45 minutes. At the end of this time, 90 µL of Substrate Solution was added, followed by a 20-minute incubation at 37 °C in the dark; then, 50 µL of Stop Solution was used to block the enzymatic reaction. The plate was analyzed on a spectrophotometer (Infinite 200 Pro MPlex, Tecan, Männedorf, Switzerland) at a wavelength of 450 nm. The concentration is expressed in pg/mL relative to a standard curve (range 0 to 1500 pg/mL) and the results are expressed as a percentage (%) compared to the control (line 0). *Figure 3* *C*

The data obtained, shown in Figures 2 and 3, show that intestinal metabolism has a physiological trend. In particular, the analysis of the intestinal epithelium demonstrates that all tested strains were able to maintain epithelial integrity compared to the control (p < 0.05), increasing the ionic flux of paracellular exchanges across the intestinal barrier. In particular, the best effect was obtained following stimulation with the combination formulated with B. bifidum GM-25 DSM 34624, B. infantis GM-21 DSM 34621 and L. rhamnosus GM-28 DSM 34619 (p < 0.05), during all times of stimulation.

The TJ analysis also confirmed the results obtained by TEER. In particular, ZO-1, which maintains and at the same time modulates the integrity of the barrier, Claudine, the main protein that forms the barrier, and Occludin, which contributes to the stabilization and optimal function of the barrier, were analyzed. The results reported in Figure 3 show that all the probiotic strains B. bifidum GM-25 DSM 34624, B. infantis GM-21 DSM 34621 and L. rhamnosus GM-28 DSM 34619 act positively on the parameters considered. The mixture of the 3 probiotics shows an improving effect *(**Figure 3**)*

### Effectiveness

### (b) Evaluation of oxidative stress and inflammation (ROS and TNFalpha)

### Oxidative stress

Considering that oxidative stress is a key factor in obesity and metabolic syndrome, the production of ROS (Reactive Oxygen Species) was also analyzed.

The degree of superoxide anion release was used to examine the ROS produced after the stimulations. After treatment, 100 µl of cytochrome C and, in another sample, 100 µl of superoxide dismutase were added for 30 minutes in an incubator. Absorbance was measured by a spectrometer, at 550 nm, and O2 was expressed as nanomoles of reduced cytochrome C per microgram of protein relative to the control and expressed as a percentage (%).

The analyzes conducted demonstrate that only B. infantis GM-21 DSM 34621 and L. rhamnosus GM-28 DSM 34619 reduce the production of ROS, suggesting the absence of damage at the intestinal barrier level. In any case, the combination formulated with B. bifidum GM-25 DSM 34624, B. infantis GM-21 DSM 3462 and L. rhamnosus GM-28 DSM 34619 amplifies the beneficial effect of the individual probiotics (p<0.05). *Figure 4*

### TNF alpha

The concentration of TNFα was determined using the TNFα ELISA kit (Merck Life Science, Rome, Italy) following the experimental protocol]. Colorimetric intensity was measured at 450 nm by spectrophotometer (Infinite 200 Pro MPlex, Tecan, Männedorf, Switzerland). The results were calculated by generating a calibration curve (range from 24.58pg/ml to 6000pg/ml) and the results were expressed as a percentage (%) compared to the control (line 0).

The data obtained on oxidative stress are also confirmed by the analysis of TNFα, which support the hypothesis of the absence of intestinal inflammation. The formulation consisting of the association of the three probiotic strains B. bifidum GM-25 DSM 34624, B. infantis GM-21 DSM 34621 and L. rhamnosus GM-28 DSM 34619 reduces the activity of the pro-inflammatory cytokine compared to the control and compared to individual strains (p<0.05). amplifying the beneficial effect of individual probiotics on inflammation *Figure 5**.*

### c. Analysis of secondary metabolites

In the intestine, butyrate produced by the intestinal microflora can play a key role in various physiological processes: in fact it is a regulator in the transport of transepithelial fluids, improving any inflammatory state of the mucosa and modulating visceral sensitivity and intestinal motility. Furthermore, it has recently been highlighted that butyrate can prevent obesity and related metabolic diseases both through endogenous production and exogenous intake of supplements as suggested by a randomized clinical trial in which oral butyrate supplementation was effective in the treatment of pediatric obesity. Considering this evidence, the production of butyric acid following stimulation with the probiotic strains under examination was analyzed. The data has highlighted how combinations of probiotics are able to stimulate the production of second messengers in the bloodstream.

Butyric acid produced at the basolateral level was analyzed by ELISA kit (Cloud-Clone, Wuhan) according to the manufacturer's instructions. The absorbance of each sample was measured after the addition of stop solution at 450 nm using a plate reader (Infinite 200 Pro MPlex, Tecan, Männedorf, Switzerland), and the measured absorbances were interpolated with a standard curve (from 0 to 10,000 pg/mL), expressing the data as the mean (pg/mL) versus control of five independent experiments performed in triplicate.

As shown in Figure 6, B. infantis GM-21 DSM 34621 was not able to induce the production of butyric acid (p>0.05), contrary to what happened for B.bifidum GM-25 DSM 34624 and L. rhamnosus GM-28 DSM 34619. The latter strain alone induces the greater production of butyric acid compared to the control (p<0.05). Also in this test the association of the three probiotics produced an improving effect.

### Example 3

### Safety and efficacy of probiotics on adipocytes

Following the evaluation of the safety and effectiveness of probiotics in the intestinal environment, further analyzes were carried out on their effect on adipocytes. As reported in *Figure 7* *A,* it is possible to observe how the tested strains induced an increase in cell viability compared to the control (p<0.05), an effect amplified when the probiotics were tested in combination.

Total lipid accumulation was assessed with the Adipogenesis Assay Kit (Cayman, Ann Arbor, MI, USA) following the recommendations indicated by the manufacturer. Briefly, after stimulation, cells were fixed in 75 µL of fixative for 15 min, washed twice with 100 µL of wash solution, stained with 75 µL of Oil Red O working solution for 20 min at room temperature, and observed under the microscope (Leica DM1000). To quantify lipid accumulation, 100 µL of dye extraction solution was added to each well, mixed gently for 30 minutes, and absorbance was measured at 490-520 nm using a plate reader (Infinite 200 Pro MPlex , Tecan, Männedorf, Switzerland). The results were obtained by comparing the absorbances of the samples with that of the control cells (baseline 0%).

To confirm the active role of probiotics in slowing down adipogenesis, the accumulation of lipids in adipose tissue was analyzed. As reported in *Figure 7B*, the presence of lipid droplets was not reduced by the probiotics taken individually; however, when they were used in combination with each other they induced a decrease in the accumulation of lipids present compared to the control and compared to the individual strains (p<0.05).

These data confirm that the use of the combinations is able to reduce lipid accumulation at the adipocyte level.

### Absence of inflammatory mechanisms following stimulation with the tested strains

### Lipid peroxidation

Several studies have shown how an increase in lipid peroxidation is associated with obesity. In the analyzes reported in *Figure 8A**,* it was observed that the individual strains did not play an important role in the reduction of perox which, when present, is minimal. The combination of the 3 strains allows the oxidation of lipids accumulated in the adipocytes to be significantly reduced. Therefore the association of these strains is truly unique, confirming the positive role of probiotics in modulating adipogenesis.

These data are confirmed by the analysis of the activity of TNFα expressed by the adipocyte. Also in this case the beneficial effect is strengthened by the use of the association of the 3 probiotic strains B. bifidum GM-25 DSM 34624, B. infantis GM- 21 DSM 3462 and L. rhamnosus GM-28 DSM 34619. *Figure 8* *B*

Therefore the combination of strains reduces oxidative damage because it prevents the oxidation of lipids accumulated in the adipocyte. This is also reflected in a better inflammatory state, as confirmed by the very significant reduction of TNFalpha in the adipocytes.

The tested substances reduce the accumulation of lipids without inducing oxidative stress at hepatic levels.

### EXAMPLE 4

### Evaluation of the action of probiotics on the hepatic compartment

Considering how the increase in body fat also influences the liver compartment by promoting systemic metabolic dysfunctions, the following parameters at the liver level were also evaluated

### A. Lipid accumulation

### B. Lipid peroxidation

### Accumulation of lipids in the liver.

The analysis was carried out following pre-treatment with oleic acid for 48 hours to mimic a condition of metabolic dysfunction in obese subjects. All the probiotics examined, both individually and in combination with each other, were able to decrease the levels of lipid accumulation induced following pre-treatment with oleic acid (p<0.05). *Figure 9A*

### Lipid peroxidation

Lipid peroxidation analysis also showed similar results. In particular, all single agents were able to significantly decrease the inflammatory mechanisms induced by oleic acid (p<0.05).

Furthermore, the beneficial effect induced by the combination formulated with *Lactoacillus rhamnosus* GM-28 DSM 34619, *Bifidobacterium bifidum* GM-25 DSM 34624 and *Bifidobacterium longum subsp* infantis GM-21 DSM 34621 was further confirmed (p<0.05). *Figure 9B*

### EXAMPLE 5

### Effect of policosanols

Since policosanols are known to exert an activity on the rebalancing of the lipid structure, the effect of these substances on some of the parameters described above was evaluated to verify a possible synergistic effect with the identified association of probiotic strains. *Figure 10* *(A, B,* C *and D)*

In particular, oxidative stress and inflammation evaluated as ROS and TNF alpha, the production of butyric acid and lipid accumulation in adipocytes were determined using the methods described in the examples previously reported.

Figure 10A shows that policosanols amplify the beneficial effect of probiotics even if when used alone they induce an increase in ROS levels compared to controls and individual strains (internal data not shown). This indicates a synergy of action with the probiotic association.

To support these data which demonstrate the absence of intestinal inflammation, the data relating to the determination of TNF alpha are shown in Figure 10B. Also in this case, policosanols in association with probiotics further reduce the activity of the pro-inflammatory cytokine exerted by the strains.

Regarding the production of secondary metabolites, in the examples given above it has been highlighted that the combination of probiotics is able to stimulate their production in the bloodstream. The presence of policosanols was shown to further increase the release of butyric acid in a statistically significant manner (*Figure 10C*)

To confirm the active role of probiotics in slowing down adipogenesis, the accumulation of lipids in adipose tissue was analyzed. As reported in Figure 10D, the probiotic strains used in combination induced a decrease in the accumulation of lipids present and a greater, statistically significant effect was appreciated using policosanols. These data therefore demonstrate that policosanols in association with the mixture of probiotic strains identified have a synergistic effect and are able to reduce lipid accumulation at the adipocyte level.

### EXAMPLE 6

### Effect of vegetable oil solvent

Once the probiotic strains and their best concentrations had been selected, their activity was evaluated even in the presence of different solvents on intestinal cells, the first target involved after human oral intake.

The effectiveness of oily solvents on some of the parameters described above was studied to verify a possible synergistic effect of these substances with the association of the probiotic strains included in the formulation.

The probiotic association consisting of *Lacticaseibacillus rhamnosus* GM-28 DSM 34619, *Bifidobacterium bifidum* GM-25 DSM 34624 and *Bifidobacterium longum subsp infantis* GM-21 DSM 34621 was prepared in 4 oily matrix solvents and it was observed that the presence of these substances amplifies the cellular viability of CaCo-2 cells compared to the use of the solvent alone (p<0.05), presupposing an increase in beneficial effects (internal data not shown). It has been highlighted that the use of Ω oil induces an increase in cell vitality compared to both solvents alone and their use with the combination of probiotics (p<0.05), demonstrating that Ω oil in particular is capable of implement the activity of probiotics at the intestinal level. For this reason, this type of oil was used in subsequent experiments.

Figure 11 (A, B and C) shows the results obtained in the experiments relating to the determination of some parameters, comparing the probiotic association solubilized in oil compared to that in normal solvent.

In particular, TNF alpha (as an index of inflammation), the production of butyric acid and lipid accumulation in adipocytes were determined using the methods described in the examples reported previously.

Figure 11A highlights that the presence of Ω oil in the formulation accentuates the beneficial effect obtained with the use of the probiotic combination. In fact, the activity of the pro-inflammatory cytokine exerted by the strains is further reduced, in a statistically significant way.

Regarding the production of secondary metabolites, it has been highlighted that the presence of omega oil causes a further stimulation of the production of butyric acid in the bloodstream compared to the probiotic association in normal solvent (Figure 11B)

The impact on the accumulation of lipids in adipose tissue was also verified for oil. As reported in Figure 11C, the probiotic strains used in combination induced a decrease in the accumulation of lipids present in the adipocytes and the presence of oil amplified this effect.

The data reported above therefore demonstrate that the addition of a solvent, specifically a food-grade vegetable oil, is able to act positively on all the parameters examined, allowing the beneficial effects observed after treatment with probiotics to be amplified.

Based on all the above results it can be concluded that the use of an oil as a solvent further improves the effectiveness of the hypothesized product consisting of probiotics, policosanols and vegetable oil.

### Formulation examples

Double-layer tablet comprising:
- in the first layer Policosanols (10 mg) and excipients (microcrystalline cellulose, Fructose, PHGG, Calcium phosphate, Flavouring, Magnesium stearate, Silicon dioxide);
- in the second layer probiotics (20 mg constituting the dosage indicated for the established daily dose) and excipients (microcrystalline cellulose, PHGG, Calcium phosphate, Magnesium stearate, Silicon dioxide)

Below are examples of posology, administration methods and dosage

### Oral administration:

Powder in sachets, sticks, tablets, capsules, drops

### Dosage:

The recommended daily dosage is preferably:
B. bifidum GM-25 0.5*10^9 UFC
B. infantis GM-21 1.0*10^9 UFC
L. rhamnosus GM-28 0.33*10^9 UFC
Policosanols 10 mg
(The policosanols that will be used will be SimbiOmega policosanols: 3% of the oil mixture (hemp flax) is added to the policosanol powder and then dried with maltodextrin or starch)
SimbiOmega Oil 0.3 mg

## Claims

1. A composition for oral use comprising, or consisting of, a mixture of at least one probiotic strain belonging to the species *Lacticaseibacillus rhamnosus,* at least one probiotic strain belonging to the species *Bifidobacterium bifidum,* and at least one probiotic strain belonging to the species *Bifidobacterium longum* subsp *infantis.*

2. The composition according to claim 1, wherein said probiotic strain belonging to the species *Lacticaseibacillus rhamnosus* is the strain *Lacticaseibacillus rhamnosus* GM-28 DSM 34619, filed at the German Collection of Microorganisms and Cell Cultures GmbH; and/or
said probiotic strain belonging to the species *Bifidobacterium bifidum* is the strain *Bifidobacterium bifidum* GM-25 DSM 34624, filed at the German Collection of Microorganisms and Cell Cultures GmbH; and/or
said probiotic strain belonging to the species *Bifidobacterium longum subsp infantis* is the strain *Bifidobacterium longum subsp infantis* GM-21 DSM 34621 filed at the German Collection of Microorganisms and Cell Culture GmbH.

3. The composition according to anyone of claims 1 or 2, wherein said probiotic strain belonging to the species *Lacticaseibacillus rhamnosus, Bifidobacterium bifidum* and/or *Bifidobacterium longum subsp infantis* is present in a concentration between 1×10⁷ and 5×10¹¹ CFU/g, preferably between 1×10⁸ and 1×10¹⁰ CFU/g.

4. The composition according to anyone of claims from 1 to 3, wherein the total concentration of probiotics is between 5×10⁷ and 5×10¹² CFU/g, preferably between 5×10⁸ and 5×10¹⁰ CFU/g.

5. The composition according to anyone of claims 1 to 3, further comprising policosanols.

6. The composition according to claim 5, wherein said policosanols are present in a concentration of from 0.1% to 80% by weight with respect to the total weight of the composition, preferably from 0.5% to 25% by weight with respect to the total weight of the composition.

7. The composition according to anyone of the previous claims, further comprising a vegetable or synthetic oil, preferably selected from olive oil, corn oil, sunflower oil, hemp oil, linseed oil or mixtures thereof.

8. The composition according to anyone of the previous claims, wherein said vegetable or synthetic oil comprises a mixture of hemp oil and linseed oil in a weight ratio of 80:20.

9. The composition according to anyone of the previous claims, wherein said vegetable or synthetic oil is present in a concentration from 1% to 20%, preferably from 1 to 10%, even more preferably 3% by weight with respect to the total weight of the composition.

10. A pharmaceutical composition, food supplement, medical device, food for special medical purposes or functional food comprising or consisting of the composition according to anyone of claims 1 to 9.

11. The composition according to anyone of the previous claims, for use in the prevention or treatment of obesity and/or metabolic syndrome.

12. A composition for oral use comprising at least one probiotic strain and policosanols, for use in the prevention or treatment of obesity and/or metabolic syndrome.

13. The composition for oral use comprising at least one probiotic strain and policosanols according to claim 12, wherein said probiotic strain belongs to the species *Lacticaseibacillus rhamnosus, Bifidobacterium bifidum* and/or *Bifidobacterium longum subsp infantis.*

14. The composition according to claim 12 or 13, wherein said policosanols are present in a concentration from 0,1% to 80% by weight with respect to the total weight of the composition, preferably from 0,5% to 25% by weight with respect to the total weight of the composition.

15. Kit of parts comprising a mixture of at least one probiotic strain belonging to the species *Lacticaseibacillus rhamnosus,* at least one probiotic strain belonging to the species *Bifidobacterium bifidum* and at least one probiotic strain belonging to the species *Bifidobacterium longum subsp infantis* as defined in any of claims 1 to 4, and policosanols.
